# EUROPEAN PATENT SPECIFICATION

(11) **EP 3 613 441 B1**
(45) Date of publication and mention of the grant of the patent: **31.05.2023**
(21) Application number: 18787253.6
(22) Date of filing: 12.04.2018
(51) Int. Cl.: A61K 51/08, A61K 103/30

(54) **DUAL-TARGET IMAGING MOLECULAR PROBE, PREPARATION METHOD THEREFOR, AND APPLICATION THEREOF**
DUAL-TARGET-BILDGEBUNGSMOLEKULARSONDE, HERSTELLUNGSVERFAHREN DAFÜR UND ANWENDUNG DAVON
SONDE MOLÉCULAIRE D'IMAGERIE À DOUBLE CIBLE, SON PROCÉDÉ DE PRÉPARATION ET APPLICATION

(30) Priority: 17.04.2017 CN 201710250652
(43) Date of publication of application: 26.02.2020
(73) Proprietor: Peking Union Medical College Hospital, Chinese Academy Of Medical Sciences, Beijing 100005 (CN)
(72) Inventor: ZHU, Zhaohui, Beijing 100005 (CN); YAO, Shaobo, Beijing 100005 (CN)
(74) Representative: Wang, Bo
(86) International application number: PCT/CN2018/082782
(87) International publication number: WO 2018/192405

(56) References cited:
- WO-A1-2013/060793
- CN-A- 106 543 268
- CN-A- 107 412 794
- Anonymous: "Dual SSTR2 and Integrin [alpha]v[beta]3 Targeting PET/CT Imaging - Full Text View - ClinicalTrials.gov", , 29 June 2016 (2016-06-29), XP055683502, Retrieved from the Internet: URL:https://clinicaltrials.gov/ct2/show/NC T02817945 [retrieved on 2020-04-06]
- BERT BERNARD ET AL: "Radiolabeled RGD-DTPA-Tyr 3 -Octreotate for Receptor-Targeted Radionuclide Therapy", CANCER BIOTHERAPY & RADIOPHARMACEUTICALS, vol. 19, no. 2, 1 January 2014 (2014-01-01), pages 173-180, XP055543504,
- K. ZHENG ET AL: "68Ga-NOTA-PRGD2 PET/CT for Integrin Imaging in Patients with Lung Cancer", THE JOURNAL OF NUCLEAR MEDICINE, vol. 56, no. 12, 1 October 2015 (2015-10-01), pages 1823-1827, XP055683727, US ISSN: 0161-5505, DOI: 10.2967/jnumed.115.160648
- HAIMING LUO ET AL: "Design and Applications of Bispecific Heterodimers: Molecular Imaging and beyond", MOLECULAR PHARMACEUTICS, vol. 11, no. 6, 2 June 2014 (2014-06-02), pages 1750-1761, XP055204892, ISSN: 1543-8384, DOI: 10.1021/mp500115x
- SHAOBO YAO ET AL: "ABSTRAC 936: PET Imaging of Lung Carcinomas with 68Ga Labeled TATE-RGD Heterodimer", THE JOURNAL OF NUCLEAR MEDICINE, vol. 58, no. 1 SUPPL., 1 May 2017 (2017-05-01), XP055543529,
- BERT, B. et al.: "Radiolabeled RGD-DTPA-Tyr3-Octreotate for Receptor-Targeted Radionuclide Therapy", Cancer Biotherapy & Radiopharmaceuticals, vol. 19, no. 2, 31 December 2004 (2004-12-31), pages 173-180, XP055543504,
- INA, I. et al.: "PET Imaging with [68Ga]NOTA-RGD for Prostate Cancer: A Comparative Study with [18F]Fluorodeoxyglucose and [ 18F]Fluoroethylcholine", Current Cancer Drug Targets, vol. 14, no. 4, 30 April 2014 (2014-04-30) , pages 371-379, XP055543516, ISSN: 1568-0096
- YAO, S.B. et al.: "ABSTRAC 936: PET Imaging of Lung Carcinomas with 68Ga Labeled TATE-RGD Heterodimer", J Nucl Med, vol. 58, no. 1 SUPPL., 1 May 2017 (2017-05-01), XP055543529,

## Description

### Technical Field

The present invention relates to a field of radioactive probes for disease diagnosis and treatment, in particular to a TATE-RGD dual-target radioactive molecular probe targeting integrin αᵥβ₃ and/or somatostatin receptor (SSTR) positive diseases and a preparation method thereof, and applications of the compound as targeting molecules for diagnosis and treatment.

### Background Art

An important part of tumor growth is neovascularization, which is regulated by a variety of protein molecules. One of the key proteins is integrin αᵥβ₃, an extracellular matrix receptor, which is a heterodimeric transmembrane glycoprotein composed of two subunits. Integrin αᵥβ₃ is one of the important molecular markers of tumors, which is highly expressed on the surface of neovascular endothelial cells and some tumor cells, such as neuroblastoma, osteosarcoma, glioblastoma, breast cancer and prostate cancer; while Integrin αᵥβ₃ is not expressed or expressed very low in formed blood vessels and normal tissues. Due to its high expression in tumor growth and metastasis, integrin αᵥβ₃ has become one of the targets for diagnosis and treatment. Somatostatin (SST) functions through the somatostatin receptor (SSTR). SSTR belongs to a family of G protein-coupled receptors and is a glycoprotein with seven transmembrane domains. There are five different molecular subtypes for SSTR gene, namely SSTR1-5. SSTR is distributed extensively. Many normal cells including endocrine cells and lymphocytes express SSTR, but its expression is more common in tumors such as gastrointestinal pancreatic neuroendocrine tumors (gastrinoma, insulinoma, glucagonoma), carcinoid, pituitary adenoma, pheochromocytoma, paraganglioma and medullary thyroid carcinoma than that in the normal tissues. SSTR plays an important role in the occurrence and development of a variety of human tumors.

SSTR is found in a variety of neuroendocrine tumors (NET), such as gastrointestinal pancreatic neuroendocrine tumors (gastrinoma, insulinoma, glucagonoma), carcinoid, pituitary adenoma, pheochromocytoma, paraganglioma, medullary thyroid carcinoma, etc. Radionuclide-labeled somatostatin analogues of ^{99m}Tc and ¹¹¹In, such as ^{99m}Tc-HYNIC-octreotide and ¹¹¹In-DTPA-octreotide, have been used in clinic for a long time and played important roles in the diagnosis and treatment of NET. In addition, the new somatostatin derivatives are also constantly emerging or improved, such as octreotide, TATE, etc., of which, TATE has a higher affinity for SSTR2 and most NETs express more SSTR2. Internationally, ⁶⁸Ga-DOTA-TATE related preclinical research has been fully carried out and ⁶⁸Ga-DOTA-TATE has been applied in clinical practice in Germany and some other countries in the world. Numerous studies have shown its superiority of the diagnosis and treatment guidance for gastrointestinal pancreatic endocrine tumor, pituitary adenoma, pheochromocytoma, paraganglioma, medullary thyroid carcinoma and small cell lung cancer, and other neuroendocrine tumors.

The arginine-glycine-aspartate (RGD) peptide-based nuclide tracer was developed in the 1980s. It can bind with the integrin αvβ3 subunit that is highly expressed on the surface of neovascular endothelial cells and tumor cells, to display the tumor and suggest the degree of tumor neovascularization. SPECT (/CT) imaging or PET (/CT) imaging with ^{99m}Tc or ¹⁸F -labeled RGD peptides has been used in clinic in Western countries such as Europe and the United States. Some initial clinical applications are also carried out in a few units in china, suggesting that it has high safety and efficacy and is valuable for the diagnosis, staging and efficacy evaluation of a variety of tumors including breast cancer, liver cancer and glioma, etc.

The positron-emitting radionuclide-labeled peptides ⁶⁸Ga-DOTA-RGD and ⁶⁸Ga-DOTA-TATE have higher sensitivity and specificity to the receptor; since integrin αᵥβ₃ and SSTR2 receptors are highly expressed in most malignant tumors and benign NET and the PET imaging has high resolution, they can quantify the absorption of tumors and organs and have better superiority than SPECT. Meanwhile, the positron-based radionuclide ⁶⁸Ga³⁺ is produced by a generator, with the advantages of simple production process and low cost, so the PET/CT imaging with ⁶⁸Ga labeled drugs is necessary and will have higher clinical application value, to improve the detection rate of tumors; in addition, it has higher clinical significance in tumor staging and prognosis evaluation, tumor-directed surgery and therapeutic evaluation.

"Dual SSTR2 and Integrin [alpha]v[beta]3 Targeting PET/CT Imaging - Full Text View - ClinicalTrials.gov", 29 June 2016, relates to an open-label positron emission tomography/computed tomography (PET/CT) study to investigate the diagnostic performance and evaluate the efficacy of 68Ga-NOTA-3PTATE-RGD in lung cancer patients and neuroendocrine neoplam patients.

BERT BERNARD ET AL., Radiolabeled RGD-DTPA-Tyr3-Octreotate for Receptor-Targeted Radionuclide Therapy. CANCER BIOTHERAPY & RADIOPHARMACEUTICALS. Vol. 19, no. 2, 1 January 2014, pages 173-180, discloses a radiopeptide for the treatment of cancers which overexpress cell surface somatostatin receptors. The new radiopharmaceutical is composed of a somatostatin receptor-targeting peptide, a chelator (DTPA) to enable radiolabeling, and an apoptosis-inducing RGD (arginine-glycine-aspartate) peptide moiety.

K. ZHENG ET AL., 68Ga-NOTA-PRGD2 PET/CT for Integrin Imaging in Patients with Lung Cancer. THE JOURNAL OF NUCLEAR MEDICINE. Vol. 56, no. 12, 1 October 2015, pages 1823-1827, ISSN: 0161-5505, DOI: 10.2967/jnumed. 115. 160648 assesses the diagnostic value of ⁶⁸Ga-NOTA-PRGD2 (NOTA-PRGD2 is NOTA-PEG4-E[c(RGDfK)]₂) PET/CT in lung cancer.

HAIMING LUO ET AL., Design and Applications of Bispecific Heterodimers: Molecular Imaging and beyond. MOLECULAR PHARMACEUTICS, vol. 11, no. 6, 2 June 2014, pages 1750-1761, ISSN: 1543-8384, DOI: 10.1021/mp500115x summarizes the design strategies for bispecific peptide- and antibody-based heterodimers and their applications in molecular targeting and imaging.

SHAOBO YAO ET AL., PET Imaging of Lung Carcinomas with 68Ga Labeled TATE-RGD heterodimer. THE JOURNAL OF NUCLEAR MEDICINE. Vol. 58, no. 1 SUPPL., 1 May 2017, discloses a TATE-RGD heterodimer which was synthesized from octreotate and RGD peptide through a glutamate and PEG linker and then labeled with ⁶⁸Ga using a 1,4,7-triazacylononane-1,4,7-triacetic acid (NOTA) chelating group.

### Summary of the Invention

The object of the present invention is to design and synthesize a TATE-RGD dual-target radioactive molecular probe and a preparation method thereof. The molecular probe contains two polypeptides of TATE and RGD for binding two targets on tumor cells, which is linked by PEG (PEG = polyethylene glycol) molecule, playing a role of adjusting the distance between functional groups and the pharmacokinetic properties in vivo. Compared with single-target molecular probes, this probe has higher tumor uptake and can achieve better in vivo imaging effect. The molecule can be radiolabeled by a bifunctional chelating agent NOTA-labeled radionuclide. In vivo, the radiolabeled probe is concentrated to the lesion by TATE and/or RGD polypeptide tumor cell receptor targeting, and perform imaging diagnosis and treatment of the lesions with highly expressed SSTR2 and/or integrin αᵥβ₃ using nucleus medical positron emission tomography.

The object of the invention is achieved by the following technical solutions:
First, the present invention provides a targeting polypeptide compound having dual targets, comprising a TATE cyclic peptide structure, an RGD cyclic peptide structure and a NOTA chelating group, wherein the TATE cyclic peptide structure and the RGD cyclic peptide structure are linked to two carboxyl terminals of the same glutamic acid molecule by a PEG₄ molecular segment respectively to form a stable amide bond; the NOTA chelating group is linked to the amino terminal of the same glutamic acid molecule by a PEG₄ segment; the polypeptide compound is designated as NOTA-3PEG₄-TATE-RGD, and the specific structure thereof is as shown in the formula (I) below:

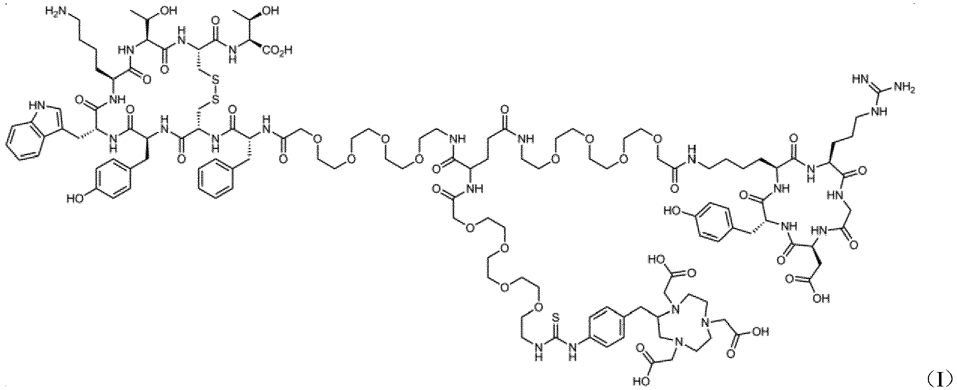

The present invention further provides a TATE-RGD dual-target radioactive molecular probe, a radionuclide-labeled polypeptide complex, wherein the polypeptide complex takes the targeting polypeptide compound having dual targets of the invention as a ligand.

In a preferred TATE-RGD dual-target radioactive molecular probe herein, the radionuclide is selected from any one of ⁶⁸Ga, ⁶⁴Cu, ¹⁸F, ⁸⁹Zr or ¹⁷⁷Lu; further preferably from any one of ⁶⁸Ga, ⁶⁴Cu or ¹⁸F.

In a preferred embodiment of the invention, the TATE-RGD dual-target radioactive molecular probe is a ⁶⁸Ga-labeled polypeptide complex, the polypeptide complex takes the targeting polypeptide compound having dual targets of the invention as a ligand, and the TATE cyclic peptide structure, the RGD cyclic peptide structure and the NOTA chelating group of the dual-target tumor targeting polypeptide compound are linked to a same glutamic acid molecule by a PEG segment having a polymerization degree of 2 to 5.

In a most preferred embodiment of the invention, the TATE-RGD dual-target radioactive molecular probe is a ⁶⁸Ga-labeled polypeptide complex, the polypeptide complex takes the targeting polypeptide compound NOTA-3PEG₄-TATE-RGD having dual targets of the invention as a ligand, and the dual-target radioactive molecular probe is simply represented as ⁶⁸Ga-NOTA-3PEG₄-TATE-RGD.

Another object of the invention is to provide a method for preparing the dual-target radioactive molecular probe, which is easy and feasible, with a high stability of product. The method is as follows:
A method of preparing TATE-RGD dual-target radioactive molecular probe, taking the preparation of ⁶⁸Ga-NOTA-3PEG₄-TATE-RGD radiopharmaceutical as an example, comprising the following steps:
dissolving the NOTA-3PEG₄-TATE-RGD in deionized water; rinsing germanium-gallium (⁶⁸Ge/⁶⁸Ga) generator into a EP tube with 5 mL of 0.1 mol/L high-purity hydrochloric acid solution, collecting 1 mL of the solution containing the highest content of radioactivity, adding 93 µL of 1.25 mol/L sodium acetate to adjust the pH of the mixture to 4-4.5; adding 20 µg of the precursor to the mixture and mix well, heat to 100 °C for 10 min; after completion of the reaction, cooling the reaction solution to room temperature, then adding 4 mL of sterile water for injection, filtering the solution to a sterile product bottle through a sterile filter membrane (0.22 µm, 13 mm).

The ⁶⁸Ga-NOTA-3PEG4-TATE-RGD radioactivity detection method, including:
analytical HPLC method, using high performance liquid chromatographic instrument (Waters, USA, Model 515 pump); UV detector (Model 486, UV absorption wavelength = 254 nm), radioactivity detector (US EG&G BERTHOLD), radioactivity meter CRC-25 PET (Capintec, USA), Waters column Nova-Pak C18 (4.6 × 150 mm, 5 µm);

Further, the analytical HPLC method in the previous step is carried out using a Waters HPLC system equipped with a Waters C18 analytical column (4.6 mm × 250 mm), HPLC gradient elution conditions: 0 min, acetonitrile/water (5/95, v/v); 5 min, acetonitrile/water (5/95, v/v); 10 min, acetonitrile/water (80/20, v/v); 15 min, acetonitrile/water (100/0, v/v); 18 min, acetonitrile/water (100/0, v/v); 20 min, acetonitrile/water (5/95, v/v), 0.1% TFA in the eluent, flow rate of 1 mL/min. From the radioactive HPLC profile, the retention time of ⁶⁸Ga-NOTA-3PEG4-TATE-RGD is 11.6 min, and the radiochemical purity is >99%.

The invention further provides the TATE-RGD dual-target molecular probe for use in the preparation of a radiopharmaceutical for use in the imaging diagnosis of a SSTR2 and/or integrin αvβ3 positive lesion.

The preferred application of the invention is that the TATE-RGD dual-target molecular probe is prepared as a colorless transparent injectable composition for small cell lung cancer imaging diagnosis.

Compared with the prior art, the TATE-RGD dual-target molecular probe has the following beneficial effects.
1. The TATE-RGD of the present invention is a dual-target polypeptide drug, and TATE and RGD are linked to enable simultaneous binding to SSTR and integrin αᵥβ₃, which increases binding affinity and tumor uptake of drugs, and achieves better tumor imaging effect;
2. In the present invention, by introducing a PEG molecule having the same or different degree of polymerization between the TATE and the RGD polypeptide, it can improve the pharmacokinetic properties of the polypeptide drug, especially the non-tumor tissue clearance rate;
3.The use of NOTA as a chelating agent in the present invention has better in vivo and in vitro stability than that of DOTA.

### Brief Description of the Drawings

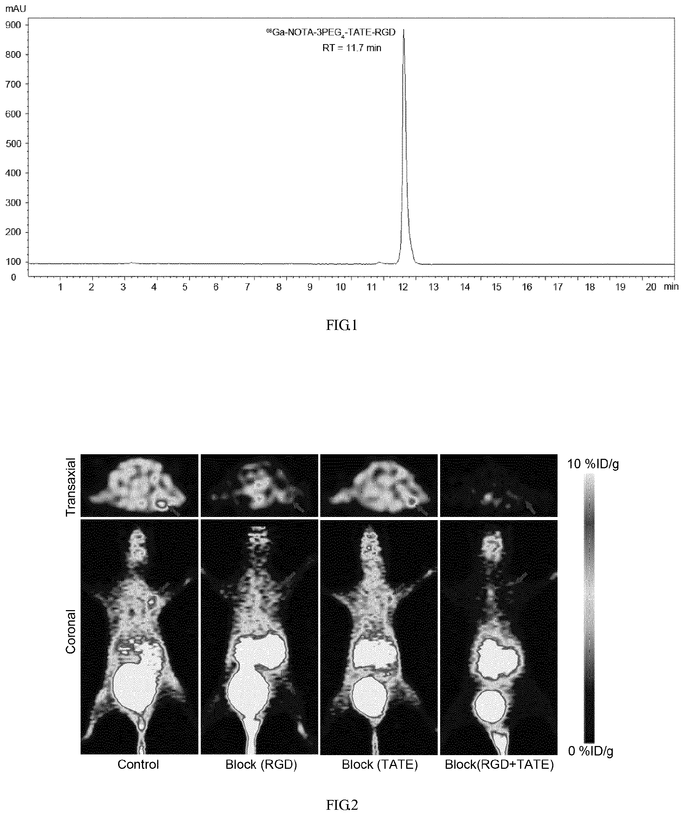

FIG. 1 shows ⁶⁸Ga-NOTA-3PEG₄-TATE-RGD HPLC pattern.
FIG.2 shows a microPET imaging result of small cell lung cancer H69 model of nude mice. From the left to the right, the imaging results of injection with ⁶⁸Ga-NOTA-3PEG₄-TATE-RGD (Control), ⁶⁸Ga-NOTA-3PEG₄-TATE-RGD+RGD (Block+RGD), ⁶⁸Ga-NOTA-3PEG₄-TATE-RGD+TATE (Block+TATE) and ⁶⁸Ga-NOTA-3PEG₄-TATE-RGD +RGD +TATE (Block+TATE+RGD) from tail veins, respectively.

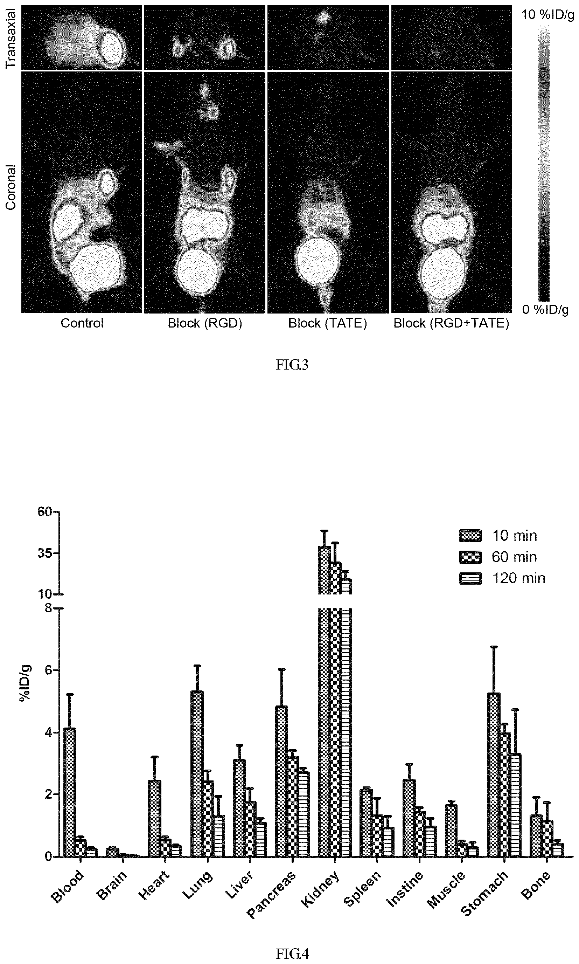

FIG.3 shows a microPET imaging result of non-small cell lung cancer A549 model of nude mice. From the left to the right, the imaging results of injection with ⁶⁸Ga-NOTA-3PEG₄-TATE-RGD (Control), ⁶⁸Ga-NOTA-3PEG₄-TATE-RGD+RGD (Block+RGD),⁶⁸Ga-NOTA-3PEG₄-TATE-RGD+TATE(Block+TATE) and ⁶⁸Ga-NOTA-3PEG₄-TATE-RGD+RGD+TATE (Block+TATE+RGD) from tail veins, respectively.
FIG. 4 shows the radioactive uptake %ID/g of each organ at different time points after injection of ⁶⁸Ga-NOTA-3PEG₄-TATE-RGD in normal mice.

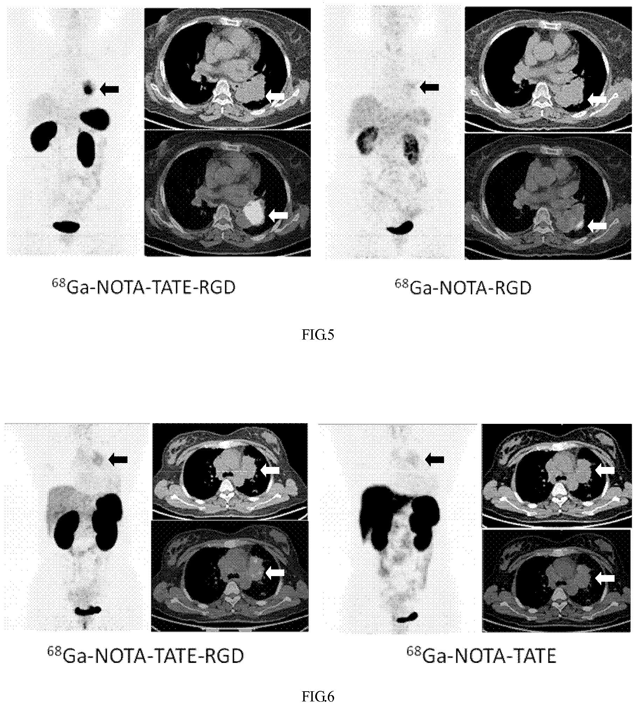

FIG. 5 shows comparison of imaging results of ⁶⁸Ga-NOTA-3PEG₄-TATE-RGD of the present invention with the existing ⁶⁸Ga-NOTA -RGD probe in patients with non-small cell lung cancer, wherein the left figure is the imaging result of ⁶⁸Ga-NOTA-3PEG₄-TATE-RGD of the invention, and the right figure is the imaging result of existing ⁶⁸Ga-NOTA-RGD, and the arrow points to the lesion.
FIG. 6 shows comparison of imaging results of ⁶⁸Ga-NOTA-3PEG₄-TATE-RGD with the existing ⁶⁸Ga-NOTA - RGD probe in patients with small cell lung cancer, wherein the left figure is the imaging result of ⁶⁸Ga-NOTA-3PEG₄-TATE-RGD of the invention, and the right figure is the imaging result of existing ⁶⁸Ga-NOTA -RGD, and the arrow points to the lesion.

### Detailed Description of the Preferred Embodiments

In order to explain the technical solutions of the present invention more clearly, the present invention will be further illustrated and described below with reference to the accompanying drawings and embodiments. The chemical products and reagents used in the following embodiments herein are existing or commercially available products.

### Embodiment 1

A targeting polypeptide compound having dual targets, comprising a TATE cyclic peptide structure, an RGD cyclic peptide structure and a NOTA chelating group, formed by linking the same glutamic acid molecule by the TATE cyclic peptide structure, the NOTA chelating group and the RGD cyclic peptide structure respectively, wherein the TATE cyclic peptide structure and the RGD cyclic peptide structure are linked to the two carboxyl terminals of the glutamic acid molecule through a PEG₄ molecular segment respectively to form a stable amide bond; the NOTA chelating group is linked to the amino terminal of the glutamic acid molecule by a PEG₄ molecule; the obtained polypeptide compound is designated as NOTA-3PEG₄-TATE-RGD, and the specific structure thereof is as shown in the formula (I) below:

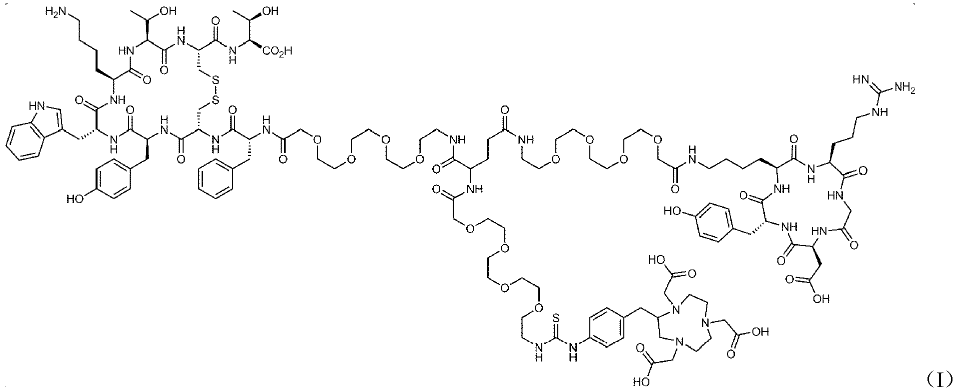

A method of preparing the NOTA-3PEG₄-TATE-RGD comprises the following steps:
1) Synthesis of compound Glu-PEG₄-TATE:
   9.5 mg of Fmoc-Glu(Boc)-OH (Fmoc and Boc protected glutamic acid), 25 µL of diisopropylethylamine (DIPEA) and 5 µL of diethylphosphonium chloride (DECP) were added to a 20 mL glass vial containing 27.5 mg PEG₄-TATE (a commercially available tumor targeting peptide dissolved in 2.6 mL dimethylformamide (DMF)), after mixed and dissolved, the mixed solution was stirred at room temperature for 2 h, then subjected to LC-MS analysis. Results showed that the Fmoc and Boc protected amino condensation polypeptide product was obtained, recorded as intermediate compound I; then 0.6 mL piperidine was added to the intermediate compound I solution and stirred at room temperature for 1h, to remove the Fmoc protecting group of intermediate compound I and give the product Glu-PEG₄-TATE. After purification by HPLC and lyophilization, about 17.5 mg of pure compound was obtained, with a yield of 68%.
2) Synthesis of compound NOTA-2PEG₄-TATE:
   17.5 mg of the compound Glu-PEG₄-TATE obtained in step 1) dissolved in 2 mL of dimethyl sulfoxide (DMSO), 20 µL DIPEA and 24.0 mg NOTA-PEG₄-NHS (1,4,7-triazacyclononane-N,N',N"-triacetic acid activated ester, 2 equivalents) were mixed and dissolved. The mixture was stirred at room temperature for 20 min and the reaction process was monitored by HPLC. After the compound Glu-PEG₄-TATE in the above step was consumed, the generation of Boc-protected NOTA-PEG₄-TATE was detected by LC-MS. Then 0.1 mL of trifluoroacetic acid (TFA) was added to remove the protecting group Boc to obtain a target compound formed by linking glutamic acid to the PEG₄ segment respectively, simply represented as NOTA-2PEG₄-TATE. The mixture obtained by lyophilization and removal of the solvent DMSO was separated and purified by HPLC, and after lyophilization, about 5.5 mg of target product was obtained, with a yield of 25.6%.
3) Synthesis of compound NOTA-3PEG₄-TATE-RGD:
   In a 20 mL glass reaction vial containing 6.5 mg of NOTA-2PEG₄-TATE (dissolved in 1 mL DMSO) obtained in the step 2), 6.0 mg PEG₄-RGD and 10 µL DIPEA were added to mix and dissolve. The mixture was stirred at room temperature for 1 h and purified by HPLC. Purification conditions: diluted with 4 mL of water and preparative HPLC (with C18 column) was applied to inject samples in two portions, and purified at a flow rate of 12 mL/min according to the following gradient. The eluent ingredients: Buffer A: 0.1% TFA in H₂O; Buffer B 0.1% TFA in CH₃CN; gradient elution: 0-20 min: 20-50% Buffer B. After lyophilization, about 1.5 mg of product was obtained, with a yield of 13.5% and a purity of more than 97%. The product was identified by LC-MS: [(MHH)/2]⁺⁺= 2930.14, and the calculated (m/z) was 2930.92 (C₁₃₁H₁₉₃N₂₇O₄₃S₃). The target product NOTA-Bn-p-SCN-PEG₄-Glu{PEG₄-Cyclo[Arg-Gly-Asp-(D-Tyr)-Lys]}-PEG₄-(D-Phe)-Cys-Tyr-(D-Trp)-Lys-Thr-Cys-Thr was determined.

### Embodiment 2

A TATE-RGD dual-target radioactive molecular probe comprising a TATE polypeptide, an RGD polypeptide and a radionuclide ⁶⁸Ga, wherein the TATE and RGD polypeptides are linked by a PEG₄ molecule to form a TATE-3PEG₄-RGD polypeptide and ⁶⁸Ga is linked by a NOTA. The TATE-RGD dual-target radioactive molecular probe is ⁶⁸Ga-NOTA-3PEG₄-TATE-RGD.

The method of preparing ⁶⁸Ga-NOTA-3PEG₄-TATE-RGD dual-target radioactive molecular probe, comprising the following steps:
(1) ⁶⁸Ga rinsing
   5 mL of 0.1 mol/L HCl was drawn by a 5 mL syringe to elute the germanium-gallium generator slowly, at the same time, the eluent was collected to a 1.5 mL EP tube, 1 mL per tube, a total of 5 tubes. Radioactivity determination was performed for each EP tube, and the most deactivated tube was used for labeling;
(2) Labeling of ⁶⁸Ga-NOTA-3PEG₄-TATE-RGD
   1.25 mol/L sodium acetate solution was added to a 1 mL⁶⁸Ga eluent, the pH of solution was adjusted to 4.0-4.5, then mixed well, and 20 µg of NOTA-3PEG₄-TATE-RGD prepared in the Embodiment 1 was added to mix well, heated to 100°C and kept for 10 min; after the reaction was completed, the reaction solution was cooled to room temperature, and 4 mL of sterile water for injection was added, and then filtered to a sterile product bottle through a sterile filter membrane (0.22 µm, 13 mm);
(3) Quality control
   HPLC conditions: C18 column (4.6 mm × 250 mm), mobile phase A: 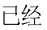 0.1% trifluoroacetic acid, mobile phase B : water (0.1% trifluoroacetic acid), and flow rate of 1 mL/min: 0~5 min, mobile phase A 5%;10 min, mobile phase A: 80%; 15 min, mobile phase A: 100%; 18 min, mobile phase A: 100%; 20 min, mobile phase A 5%. The UV detection wavelength was 210 nm and the column temperature was 20 °C. Radioactive detection was performed by a dedicated radioactive detector for HPLC. HPLC analysis showed that the retention time of ⁶⁸Ga-NOTA-3PEG₄-TATE-RGD was 11.6 min (FIG. 1), and the radiochemical purity was >99%, without requiring further purification.
(4) Determination of in vitro stability
   After ⁶⁸Ga-NOTA-3PEG₄-TATE-RGD labeling, fetal bovine serum was added and the radiochemical purity was determined by HPLC at 60 and 120 min, respectively. After determination, the radiochemical purity was 98% and 97%, respectively.

The above TATE-RGD dual-target probe ⁶⁸Ga-NOTA-3PEG₄-TATE-RGD was further prepared into a colorless transparent injection for imaging test or imaging diagnosis. The specific experiments and effects were as follows:

### ① microPET imaging of small cell lung cancer H69 tumor-bearing mice

The tumor-bearing mice inoculated with H69 tumor subcutaneously in the right forelimb were injected with ⁶⁸Ga-NOTA-3PEG₄-TATE-RGD injection in Embodiment 2 by tail veins at 100-200 µCi for 60 min, and then 10 min static image was collected by a Siemens Inveon micro PET (FIG. 2, wherein the arrow indicated the tumor site). The results showed that, when the probe of Embodiment 2 was injected alone, there was significant radioactivity uptake at the tumor site, the tumor was clearly visible, and the tumor uptake value was 9.78 ± 2.77; while the co-injection of the unlabeled precursor RGD, TATE and RGD+TATE was effective in reducing tumor uptake to 8.23 ± 1.08, 1.41 ± 0.73, and 1.05 ± 0.13, respectively; when concurrently injected with unlabeled precursors RGD and TATE, the radioactivity uptake of tumor was nearly reduced to the background. Small cell H69 tumors were predominantly expressed by SSTR, and the concentration of the dual-target molecular probe of the present invention in tumors was mainly inhibited by unlabeled TATE.

### (2) microPET imaging of non-small cell lung cancer A549 tumor-bearing mice

The tumor-bearing mice inoculated with A549 tumors in the right forelimb were injected with ⁶⁸Ga-NOTA-3PEG₄-TATE-RGD of injection of embodiment 2 by tail veins at 100-200 µCi for 60 min, and then 10 min static image was collected by a Siemens Inveon micro PET (FIG. 3, wherein the arrow indicated the tumor site). The results showed that, when the probe of Embodiment 2 was injected alone, there was significant radioactivity uptake at the tumor site, the tumor was clearly visible, and the tumor uptake value was 6.46 ± 0.59; while the co-injection of the unlabeled precursor RGD, TATE and RGD+TATE was effective in reducing tumor uptake to 1.75 ± 0.53, 3.80 ± 0.48 and 1.35 ± 0.26, respectively; when concurrently injected with unlabeled precursors RGD and TATE, the radioactivity uptake of tumor was nearly reduced to the background. Non-small cell A549 tumors were predominantly expressed by integrin receptor, and the concentration of the dual-target molecular probe of the present invention in tumors was mainly inhibited by unlabeled RGD.

### ④Biodistribution of ⁶⁸Ga-NOTA-3PEG₄-TATE-RGD in healthy mice

The distribution of healthy Balb/c was shown in FIG. 4, and ⁶⁸Ga-NOTA-3PEG₄-TATE-RGD of Embodiment 2 was cleared from the blood, heart and liver faster; the kidney was more radioactive, indicating that the molecular probe was mainly excreted through the kidneys. There was small amount of uptake in the heart, liver and lungs, which decreased quickly over time. The imaging agent had a small amount of distribution in the stomach, intestines, spleen, and pancreas, and little distribution in the brain tissues, indicating that it could not pass through the blood-brain barrier.

### ⑤Imaging of ⁶⁸Ga-NOTA-3PEG₄-TATE-RGD in patients with small cell lung cancer

The ⁶⁸Ga-NOTA-3PEG₄-TATE-RGD PET/CT images of clinically diagnosed small cell lung cancer patients were shown in FIG. 5. 68Ga-NOTA-3PEG₄-TATE-RGD 3.5 mCi was injected intravenously, 30 min later, the images of truck were collected by Siemens Biograph64 PET/CT, 3 min/bed, and a total of 5 beds were collected. The lesions were clearly displayed and the highest standard uptake value of tumor (SUVmax) was 18.2 (FIG. 5 left). However, the existing single-target imaging agent only showed an increase in the ⁶⁸Ga-NOTA-RGD uptake of the tumor region, and the SUVmax value was 4.7 (FIG. 5 right).

### ⑥Imaging of ⁶⁸Ga-NOTA-3PEG₄-TATE-RGD in patients with non- small cell lung cancer

The images of clinically diagnosed non-small cell lung cancer patients were shown in FIG. 6. 68Ga-NOTA-3PEG₄-TATE-RGD 3.0 mCi was injected intravenously, 30 min later, the images were collected by Siemens Biograph64 PET/CT, 3 min/bed, and a total of 5 beds were collected. The lesions were clearly displayed and the highest standard uptake value of tumor (SUVmax) was 3.4 (FIG. 6 left). However, the existing single-target imaging agent showed low tumor uptake of ⁶⁸Ga-NOTA-RGD, and the SUVmax value was 2.8 (FIG. 6 right).

## Claims

1. A targeting polypeptide compound having dual targets, comprising a TATE cyclic peptide structure, an RGD cyclic peptide structure and a NOTA chelating group, wherein the TATE cyclic peptide structure and the RGD cyclic peptide structure are linked to two carboxyl terminals of the same glutamic acid molecule by a PEG₄ molecular segment respectively to form a stable amide bond; the NOTA chelating group is linked to the amino terminal of the same glutamic acid molecule by a PEG₄ segment; the polypeptide compound is designated as NOTA-3PEG₄-TATE-RGD, and the specific structure thereof is as shown in the formula (I) below:

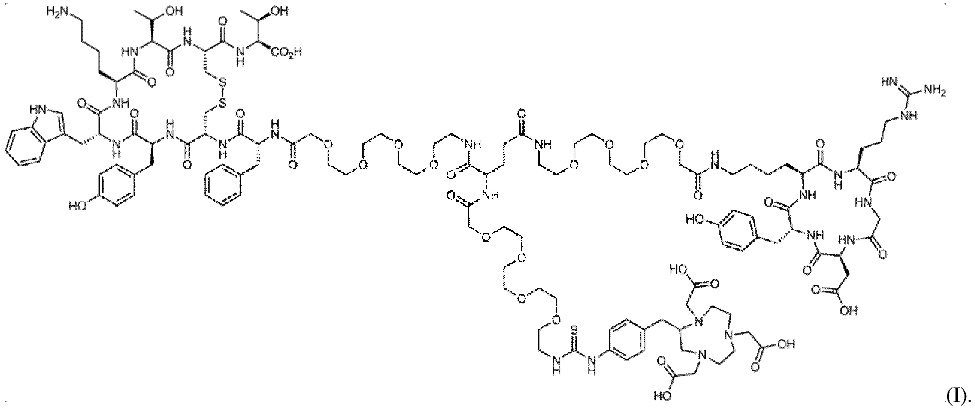

2. A TATE-RGD dual-target radioactive molecular probe, which is a radionuclide-labeled polypeptide complex, wherein the polypeptide complex takes the targeting polypeptide compound having dual targets of claim 1 as a ligand.

3. The TATE-RGD dual-target radioactive molecular probe of claim 2, wherein the radionuclide is selected from any one of ⁶⁸Ga, ⁶⁴Cu, ¹⁸F, ⁸⁹Zr or ¹⁷⁷Lu; preferably from any one of ⁶⁸Ga, ⁶⁴Cu, or ¹⁸F; and most preferably ⁶⁸Ga.

4. The TATE-RGD dual-target radioactive molecular probe of claim 2, wherein it is a radionuclide ⁶⁸Ga-labeled polypeptide complex, the polypeptide complex takes the targeting polypeptide compound having dual targets of claim 1 as a ligand, the specific structure of the targeting polypeptide compound being as shown in the formula (I) below: 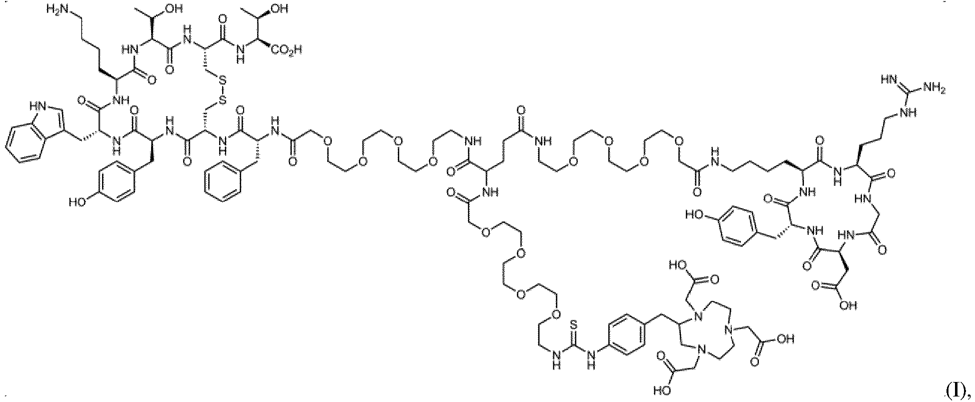 and the dual-target radioactive molecular probe is simply represented as ⁶⁸Ga-NOTA-3PEG₄-TATE-RGD.

5. A method of preparing dual- target radioactive molecular probe of claim 4, comprising the following steps:
dissolving the NOTA-3PEG₄-TATE-RGD of claim 1 in deionized water; rinsing germanium-gallium (⁶⁸Ge/⁶⁸Ga) generator into a EP tube with 5 mL of 0.1 mol/L high-purity hydrochloric acid solution, collecting 1 mL of the solution containing the highest content of radioactivity, adding 93 µL of 1.25 mol/L sodium acetate to adjust the pH of the mixture to 4-4.5; adding 20 µg of the precursor to the mixture and mix well, heat to 100 °C for 10 min; after completion of the reaction, cooling the reaction solution to room temperature, then adding 4 mL of sterile water for injection, filtering the solution to a sterile product bottle through a sterile filter membrane (0.22 µm, 13 mm).

6. Use of the targeting polypeptide compound having dual targets of claim 1 for the preparation of radiopharmaceuticals for the imaging diagnosis of a SSTR2 and/or integrin αvβ3 positive lesion.

7. The TATE RGD dual-target radioactive molecular probe of claim 2 for use in the imaging diagnosis of a SSTR2 and/ or integrin αvβ3 positive lesion; preferably the TATE-RGD dual-target molecular probe of claim 2 for use as a colorless transparent injectable composition for small cell lung cancer imaging diagnosis.

## Patentansprüche

1. Targeting-Polypeptidverbindung mit dualen Targets, umfassend eine zyklische TATE-Peptidstruktur, eine zyklische RGD-Peptidstruktur und eine NOTA-Chelatgruppe, wobei die zyklische TATE-Peptidstruktur und die zyklische RGD-Peptidstruktur jeweils durch ein PEG4-Molekülsegment an zwei Carboxylenden desselben Glutaminsäuremoleküls gebunden sind, um eine stabile Amidbindung zu bilden; die NOTA-Chelatgruppe durch ein PEG4-Segment an das Aminoende desselben Glutaminsäuremoleküls gebunden ist; die Polypeptidverbindung mit NOTA-3PEG4-TATE-RGD bezeichnet wird, und ihre spezifische Struktur wie in der nachstehenden Formel (I) gezeigt ist:

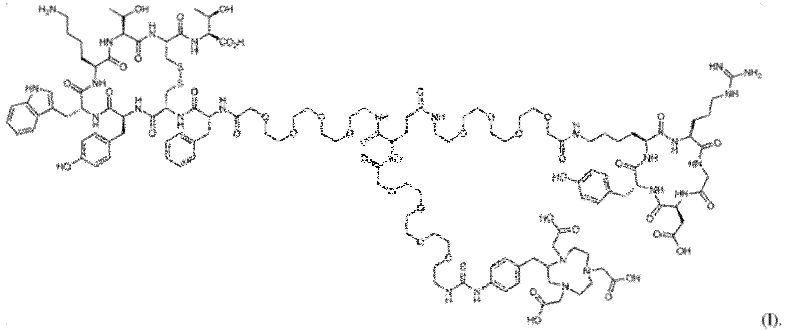

2. Radioaktive molekulare TATE-RGD-Dual-Target-Sonde, die ein mit Radionuklid markierter Polypeptidkomplex ist, wobei der Polypeptidkomplex die Targeting-Polypeptidverbindung, die duale Targets nach Anspruch 1 aufweist, als Liganden nimmt.

3. Radioaktive molekulare TATE-RGD-Dual-Target-Sonde nach Anspruch 2, wobei das Radionuklid ausgewählt ist aus einem von ⁶⁸Ga, ⁶⁴Cu, ¹⁸F, ⁸⁹Zr oder ¹⁷⁷Lu; vorzugsweise aus einem von ⁶⁸Ga, ⁶⁴Cu oder ¹⁸F; und besonders bevorzugt ⁶⁸Ga.

4. Radioaktive molekulare TATE-RGD-Dual-Target-Sonde nach Anspruch 2, wobei sie ein mit Radionuklid ⁶⁸Ga-markierter Polypeptidkomplex ist, wobei der Polypeptidkomplex die Targeting-Polypeptidverbindung, die duale Targets nach Anspruch 1 aufweist, als Liganden nimmt, wobei die spezifische Struktur der Targeting-Polypeptidverbindung wie in der nachstehenden Formel (I) gezeigt ist: 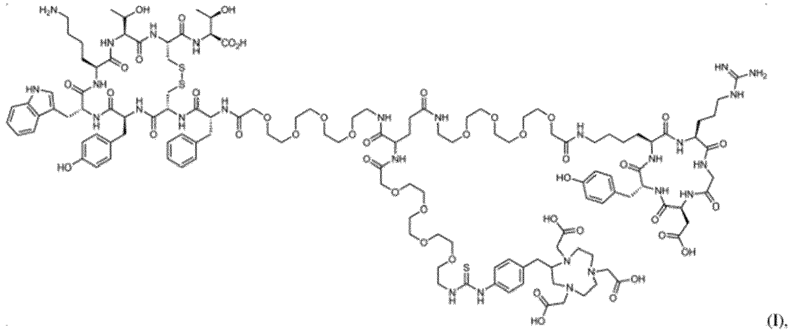 und die radioaktive molekulare Dual-Target-Sonde einfach als ⁶⁸Ga-NOTA-3PEG4-TATE-RGD dargestellt ist.

5. Verfahren zur Herstellung einer radioaktiven molekularen Dual-Target-Sonde nach Anspruch 4, umfassend die folgenden Schritte:
Auflösen des NOTA-3PEG4-TATE-RGD nach Anspruch 1 in deionisiertem Wasser; Spülen des Germanium-Gallium (⁶⁸Ge/⁶⁸Ga)-Generators in ein EP-Röhrchen mit 5 mL hochreiner 0,1 mol/L Salzsäurelösung, Auffangen von 1 mL der Lösung, die den höchsten Gehalt an Radioaktivität enthält, Hinzufügen von 93 µL 1,25 mol/L Natriumacetat, um den pH-Wert des Gemischs auf 4-4,5 anzupassen; Hinzufügen von 20 µg des Vorläufers zum Gemisch und gründliches Mischen, Erwärmen auf 100 °C für 10 min; nach Abschluss der Umsetzung Abkühlen der Reaktionslösung auf Raumtemperatur, dann Hinzufügen von 4 mL sterilem Wasser für die Injektion, Filtrieren der Lösung in eine sterile Produktflasche durch eine sterile Filtermembran (0,22 µm, 13 mm).

6. Verwendung der Targeting-Polypeptidverbindung mit dualen Targets nach Anspruch 1 zur Herstellung von Radiopharmazeutika für die bildgebende Diagnose einer SSTR2- und/oder Integrin-αVβ3-positiven Läsion.

7. Radioaktive molekulare TATE-RGD-Dual-Target-Sonde nach Anspruch 2 zur Verwendung bei der bildgebenden Diagnose einer SSTR2- und/oder Integrin-αvβ3-positiven Läsion; vorzugsweise die molekulare TATE-RGD-Dual-Target-Sonde nach Anspruch 2 zur Verwendung als farblose transparente injizierbare Zusammensetzung für die bildgebende Diagnose von kleinzelligem Lungenkrebs.

## Revendications

1. Composé polypeptidique de ciblage présentant des cibles doubles, comprenant une structure peptidique cyclique TATE, une structure peptidique cyclique RGD et un groupe chélatant NOTA, dans lequel la structure peptidique cyclique TATE et la structure peptidique cyclique RGD sont liées à deux terminaisons carboxyle de la même molécule d'acide glutamique par un segment moléculaire PEG₄ respectivement pour former une liaison amide stable ; le groupe chélatant NOTA est lié à la terminaison amino de la même molécule d'acide glutamique par un segment PEG₄; le composé polypeptidique est désigné par NOTA-3PEG₄-TATE-RGD, et la structure spécifique de celui-ci est telle que représentée dans la formule (I) ci-dessous :

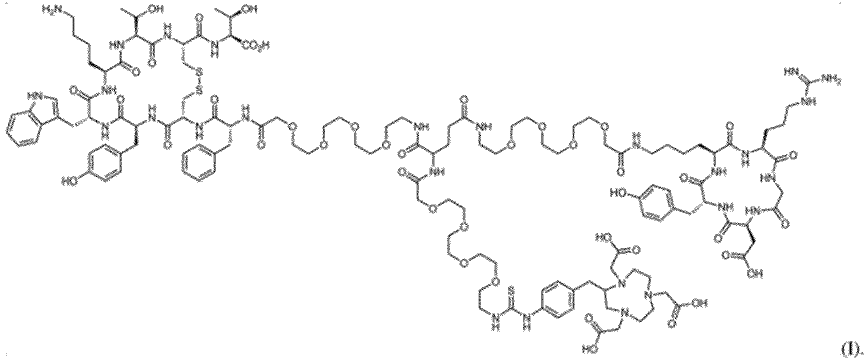

2. Sonde moléculaire radioactive à cibles doubles TATE-RGD, qui est un complexe polypeptidique marqué par un radionucléide, dans laquelle le complexe polypeptidique prend le composé polypeptidique de ciblage présentant des cibles doubles selon la revendication 1 comme ligand.

3. Sonde moléculaire radioactive à cibles doubles TATE-RGD selon la revendication 2, dans laquelle le radionucléide est choisi parmi l'un quelconque de ⁶⁸Ga, ⁶⁴Cu, ¹⁸F, ⁸⁹Zr ou ¹⁷⁷Lu ; de préférence parmi l'un quelconque de ⁶⁸Ga, ⁶⁴Cu ou ¹⁸F ; et est le plus préférentiellement Ga.

4. Sonde moléculaire radioactive à cibles doubles TATE-RGD selon la revendication 2, dans laquelle il s'agit d'un complexe polypeptidique radionucléide marqué au ⁶⁸Ga, le complexe polypeptidique prend le composé polypeptidique de ciblage présentant des cibles doubles selon la revendication 1 comme ligand, la structure spécifique du composé polypeptidique de ciblage étant telle que représentée dans la formule (I) ci-dessous : 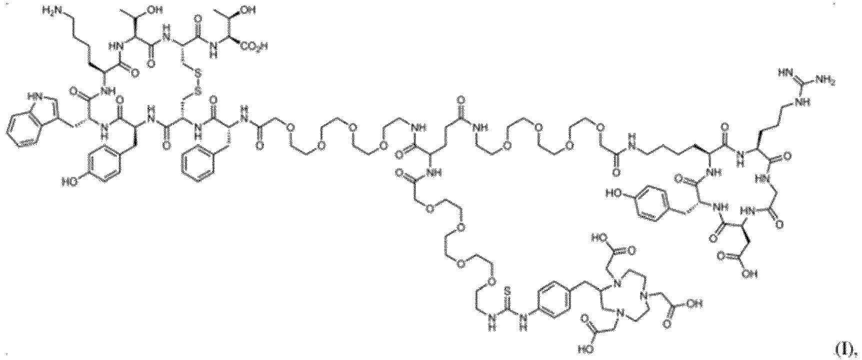 et la sonde moléculaire radioactive à cibles doubles est simplement représentée par ⁶⁸Ga-NOTA-3PEG₄-TATE-RGD.

5. Procédé de préparation d'une sonde moléculaire radioactive à cibles doubles selon la revendication 4, comprenant les étapes suivantes :
la dissolution du NOTA-3PEG₄-TATE-RGD selon la revendication 1 dans de l'eau déminéralisée ; le rinçage du générateur de germanium-gallium (⁶⁸Ge/⁶⁸Ga) dans un tube EP avec 5 ml de solution d'acide chlorhydrique de haute pureté à 0,1 mol/l, la collecte de 1 ml de la solution contenant la plus haute teneur en radioactivité, l'ajout de 93 µl de 1,25 mol/l d'acétate de sodium pour ajuster le pH du mélange à 4-4,5 ; l'ajout de 20 µg du précurseur au mélange en mélangeant avec soin, le chauffage à 100°C pendant 10 min ; une fois la réaction terminée, le refroidissement de la solution de réaction à température ambiante, puis l'ajout de 4 ml d'eau stérile pour injection, le filtrage de la solution vers un flacon de produit stérile à travers une membrane filtrante stérile (0,22 µm, 13 mm).

6. Utilisation du composé polypeptidique de ciblage présentant des cibles doubles selon la revendication 1 pour la préparation de produits radiopharmaceutiques pour le diagnostic par imagerie d'une lésion positive pour le SSTR2 et/ou l'intégrine αvβ3.

7. Sonde moléculaire radioactive à cibles doubles TATE RGD selon la revendication 2, destinée à être utilisée dans le diagnostic par imagerie d'une lésion positive pour le SSTR2 et/ou l'intégrine αvβ3; de préférence, sonde moléculaire à cibles doubles TATE-RGD selon la revendication 2 destinée à être utilisée en tant que composition injectable transparente incolore pour le diagnostic par imagerie d'un cancer du poumon à petites cellules.
